# EUROPEAN PATENT APPLICATION

(11) **EP 2 784 506 A1**
(43) Date of publication of application: **01.10.2014**
(21) Application number: 14158676.8
(22) Date of filing: 10.03.2014
(51) Int. Cl.: G01N 33/487

(54) **Measurement system**

(30) Priority: 29.03.2013 JP 2013074215; 28.02.2014 JP 2014037809
(71) Applicant: ARKRAY, Inc., Kyoto-shi, Kyoto 601-8045 (JP)
(72) Inventor: Wada, Atsushi, Kyoto-shi, Kyoto 6020008 (JP); Tanaka, Kenta, Kyoto-shi, Kyoto 6020008 (JP)
(74) Representative: MacDougall, Alan John Shaw

(57) **Abstract**

A measurement system includes a measurement device and a multifunctional portable terminal. The a measurement device includes a measurement unit for measuring a particular component in a biological sample, a measurement-side communication unit for transmitting by radio a measurement datum obtained by the measurement unit, and a measurement-side controller for controlling the measurement unit and the measurement-side communication unit. The multifunctional portable terminal includes an output-side communication unit for receiving the measurement datum transmitted from the measurement-side communication unit, an output-side storage unit for storing a plurality of voice message data, an output-side controller for generating a voice message datum or selecting a voice message datum from the voice message data stored in the output-side storage unit based on the measurement datum, and a voice message output unit for outputting the voice message datum generated or selected by the output-side controller.

## Description

### BACKGROUND OF THE INVENTION

### 1. Field of the Invention:

The present invention relates to a measurement system for e.g. self-monitoring of a blood glucose level.

### 2. Description of the Related Art:

Patients with e.g. a lifestyle-related disease regularly need to know the level (such as concentration) of a particular biological component related to the disease in order to know the state of the disease or to take proper medication. Generally, portable measurement devices are used to check the state of the biological component. For instance, a patient with diabetes needs to know his or her own blood glucose level for dietary control or proper medication. Thus, the patient needs to measure the blood glucose level regularly by carrying a device for self-monitoring of blood glucose (hereinafter referred to as an "SMBG device").

An example of an SMBG device is disclosed in JP-A-2011-247884. The SMBG device disclosed in this document includes a voice message output unit for outputting, after measurement of a blood glucose level, the blood glucose level as the measurement result by a voice message. By using this SMBG device, the patient can know the blood glucose level not only by looking at the measurement result displayed on the display unit such as a liquid crystal displaybut also by hearing the voice message from the voice message output unit.

Japanese Patent No. 4395146 discloses an SMBG device provided with a separately prepared voice message output device. The SMBG device disclosed in this document is made up of a main unit for performing blood glucose level measurement and a voice message output device capable of performing radio communication with the main unit by using e.g. infrared. In the main unit, after a blood glucose level is measured, the voice message corresponding to the measured blood glucose level is selected or generated. Then, the main unit transmits the selected or generated voice message to the voice message output device by radio. The voice message output device generally comprises an amplifier for amplifying received audio electric signals and a speaker for outputting the audio electric signals from the amplifier. By using an SMBG device of this type, the user can know the blood glucose level by the voice message from the voice message output device. By carrying the voice message output device at a position suitable for hearing, the user can reliably catch the voice message to know the blood glucose level.

According to the structure of the above-described SMBG devices, the main unit needs to have a function to perform measurement and a function to generate a voice message. While the measurement function is essential for the SMBG device, the voice message generating function is provided for the purpose of improving the convenience of the patient. When the voice message generating function is poor, the purpose of improving the convenience of the patient is not sufficiently achieved. In order that the user can easily and reliably know the blood glucose level to know his or her own condition and continue proper measurement, a versatile voice message output function is required. However, to provide a versatile voice message output function, the main unit, which has the measurement function as well, needs to be made large and complicated, and the manufacturing cost inevitably increases.

### SUMMARY OF THE INVENTION

The present invention has been proposed under the circumstances described above. It is therefore an object of the present invention to provide a measurement system that is capable of versatile voice message output while having a simple structure.

According to a first aspect of the present invention, there is provided a measurement system provided with: a measurement device including a measurement unit for measuring a particular component in a biological sample, a measurement-side communication unit for transmitting by radio a measurement datum obtained by the measurement unit, and a measurement-side controller for controlling the measurement unit and the measurement-side communication unit; and a multifunctional portable terminal including an output-side communication unit for receiving the measurement datum transmitted from the measurement-side communication unit, an output-side storage unit for storing a plurality of voice message data, an output-side controller for generating a voice message datum or selecting a voice message datum from the voice message data stored in the output-side storage unit based on the measurement datum, and a voice message output unit for outputting the voice message datum generated or selected by the output-side controller.

According to a second aspect of the present invention, in the measurement system of the first aspect, the measurement device includes a measurement-side storage unit for storing a plurality of measurement data obtained by the measurement unit.

According to a third aspect of the present invention, in the measurement system of the second aspect, the output-side controller selects or generates, as the measurement voice message datum, a measurement voice message datum including a numerical value representing the concentration of the particular component expressed in a predetermined unit, and the output-side controller causes the voice message output unit to output the measurement voice message datum.

According to a fourth aspect of the present invention, in the measurement system of the second or third aspect, the output-side storage unit stores a threshold datum to be compared with the measurement datum, and a plurality of threshold comparison result voice message data corresponding to results of comparison between the measurement datum and the threshold datum, and the output-side controller causes the voice message output unit to output one of the threshold comparison result voice message data which is selected based on a result of comparison between the measurement datum and the threshold datum.

According to a fifth aspect of the present invention, in the measurement system of the second or third aspect, the measurement-side storage unit stores a threshold datum to be compared with the measurement datum, the measurement-side controller causes the measurement-side communication unit to transmit a comparison result flag generated based on a result of comparison between the measurement datum and the threshold datum, the output-side storage unit stores a plurality of threshold comparison result voice message data corresponding to a plurality of comparison result flags, and the output-side controller causes the voice message output unit to output one of the threshold comparison result voice message data which corresponds to the comparison result flag transmitted from the measurement-side communication unit.

According to a sixth aspect of the present invention, in the measurement system of one of the first through fifth aspects, the measurement device includes a measurement-side date/time counting unit for counting date and time, the measurement-side controller obtains from the measurement-side date/time counting unit date and time at which the measurement datum is obtained to generate a measurement date/time datum, and the measurement date/time datum is stored in the measurement-side storage unit.

According to a seventh aspect of the present invention, in the measurement system of the sixth aspect, the multifunctional portable terminal includes an output-side date/time counting unit for counting date and time, the output-side communication unit is capable of performing communication using a public network, the output-side controller corrects a count of the output-side date/time counting unit based on a reference date/time datum which the output-side communication unit obtains via the public network, the output-side controller compares the measurement date/time datum transmitted from the measurement device with a date/time datum counted by the output-side date counting unit and causes the output-side communication unit to transmit a date/time correction datum when an error exceeding a predetermined level is observed in the measurement-side date/time counting unit, and the measurement-side controller corrects a count of the measurement-side date/time counting unit based on the date/time correction datum received by the measurement-side communication unit.

According to an eighth aspect of the present invention, in the measurement system of the sixth or seventh aspect, the measurement-side controller transmits a plurality of measurement data and a plurality of measurement date/time data corresponding to the measurement data, and the output-side controller generates or selects a measurement voice message datum corresponding to latest one of the plurality of measurement data received by the output-side communication unit and causes the voice message output unit to output the measurement voice message datum.

According to a ninth aspect of the present invention, in the measurement system of one of the sixth through eighth aspects, the output-side storage unit stores a plurality of measurement time period voice message data corresponding to a plurality of measurement date/time data, and the output-side controller selects one of the measurement time period voice message data which corresponds to the measurement date/time datum and causes the voice message output unit to output the measurement date/time datum.

According to a tenth aspect of the present invention, in the measurement system of one of the sixth through ninth aspects, the output-side storage unit stores a plurality of historical comparison result voice message data corresponding to results of comparison between a measurement datum and a plurality of measurement data obtained in the past, and the output-side controller causes the voice message output unit to output one of the historical comparison result voice message data which corresponds to a result of comparison between a measurement datum received by the output-side communication unit and the plurality of measurement data obtained in the past and stored in the output-side storage unit.

According to a eleventh aspect of the present invention, in the measurement system of one of the sixth through tenth aspects, the output-side storage unit stores a plurality of frequency voice message data corresponding to frequencies of measurement, and the output-side controller selects one of the frequency voice message data based on a result of comparison between a measurement date/time datum related to a newly obtained measurement datum and latest one of the measurement date/time data stored in the output-side storage unit, and causes the voice message output unit to output the frequency voice message data.

According to a twelfth aspect of the present invention, in the measurement system of one of the sixth through eleventh aspects, the output-side controller computes an average of a plurality of measurement data obtained in the past and stored at least in the output-side storage unit, selects or generates a measurement voice message datum corresponding to the average, and causes the voice message output unit to output the measurement voice message datum.

According to a thirteenth aspect of the present invention, in the measurement system of one of the first through twelfth aspects, the measurement device includes a temperature measuring unit, the measurement-side controller causes the measurement-side communication unit to transmit a measured temperature flag generated based on a temperature measured by the temperature measuring unit, the output-side storage unit stores a plurality of measured temperature voice message data corresponding to a plurality of measured temperature flags, the output-side controller causes the voice message output unit to output one of the measured temperature voice message data which corresponds to the measured temperature flag transmitted from the measurement-side communication unit.

According to a fourteenth aspect of the present invention, in the measurement system of the thirteenth aspect, the output-side storage unit stores an improper-environment voice message datum for indicating that environment in which the measurement device is used is improper, and the output-side controller causes the voice message output unit to output the improper-environment voice message datum depending on content of the measured temperature flag or the frequency.

According to a fifteenth aspect of the present invention, in the measurement system of one of the first through fourteenth aspects, the multifunctional portable terminal includes a positional data obtaining unit for obtaining positional information, the output-side storage unit stores an allowable area datum representing an area where voice message output is allowable and an inhibited area datum representing an area where voice message output should be inhibited, the output-side controller allows or inhibits voice message output by the voice message output unit based on a result of comparison between the positional information obtained by the positional information obtaining unit and the allowable area datum or the inhibited area datum.

According to a sixteenth aspect of the present invention, in the measurement system of one of the first through fifteenth aspects, the measurement system is a blood glucose level measuring system, the biological sample is blood and the particular component is glucose.

According to an embodiment of the present invention, the measurement device may only have a function of measuring a blood glucose level and transmitting the measurement value, and the generation of voice message data related to the measurement value is performed by the multifunctional portable terminal. Various kinds of voice messages can be produced by e.g., improving the program to be executed by the multifunctional portable terminal, while the measurement device does not need to have a complicated structure. In this way, a measurement system is provided that is capable of versatile voice message output while having a simple structure.

Other features and advantages of the present invention will become more apparent from detailed description given below with reference to the accompanying drawings.

### BRIEF DESCRIPTION OF THE DRAWINGS

Fig. 1 is a schematic perspective view illustrating an example of measurement system according to the present invention;
Fig. 2 is a system structural view of a measurement device of the measurement system shown in Fig. 1;
Fig. 3 is a system structural view of a multifunctional portable terminal of the measurement system shown in Fig. 1;
Fig. 4 is a flow chart of a process P01 in the measurement system of Fig. 1;
Fig. 5 is a flow chart of a process P02 in the measurement system of Fig. 1;
Fig. 6 is a flow chart of a process P03 in the measurement system of Fig. 1;
Fig. 7 is a flow chart of a process P04 in the measurement system of Fig. 1;
Fig. 8 is a flow chart of a process P05 in the measurement system of Fig. 1;
Fig. 9 is a flow chart of a process P06 in the measurement system of Fig. 1;
Fig. 10 is a flow chart of a process P07 in the measurement system of Fig. 1;
Fig. 11 is a flow chart of a process P08 in the measurement system of Fig. 1;
Fig. 12 is a flow chart of a process P09 in the measurement system of Fig. 1;
Fig. 13 is a flow chart of a process P10 in the measurement system of Fig. 1; and
Fig. 14 is a flow chart of a process P11 in the measurement system of Fig. 1.

### DESCRIPTION OF THE PREFERRED EMBODIMENTS

Preferred embodiments of the present invention are described below with reference to the accompanying drawings.

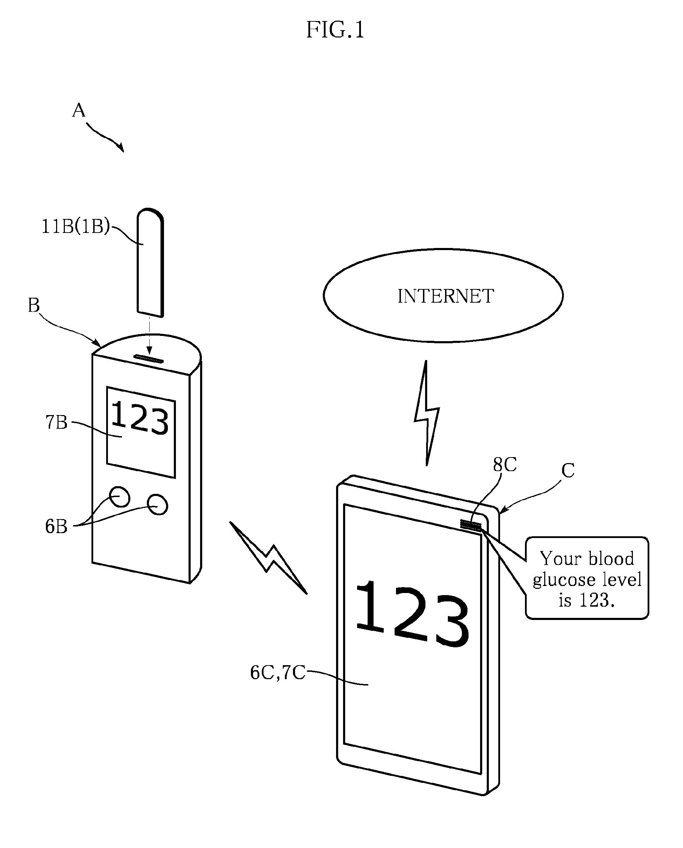

Fig. 1 illustrates an example of a measurement system according to the present invention. The measurement system A of this embodiment is made up of a measurement device B and a multifunctional portable terminal C.

The measurement device B has a measurement function to measure the concentration of a particular component in a biological sample and typically a function to measure a blood glucose level by measuring the glucose concentration in blood. The biological sample is not limited to a particular one and may be e.g. blood, urine or sweat, which can be easily taken by the user. The particular component refers to a component of which level, such as the concentration in a biological sample, is related to health condition. The health condition refers to e.g. a disease or weariness from workout. Specifically, examples of the particular component include glucose, cholesterol and triglyceride which are related to lifestyle-related diseases including diabetes, obesity or hypercholesterolemia, and lactic acid which is related to weariness from workout. For instance, a diabetes patient may check the level of glucose as the particular component. An athlete may check the level of lactic acid as the particular component. A patient with hypercholesterolemia may check the level of cholesterol as the particular component. A patient with obesity may check the level of triglyceride as the particular component.

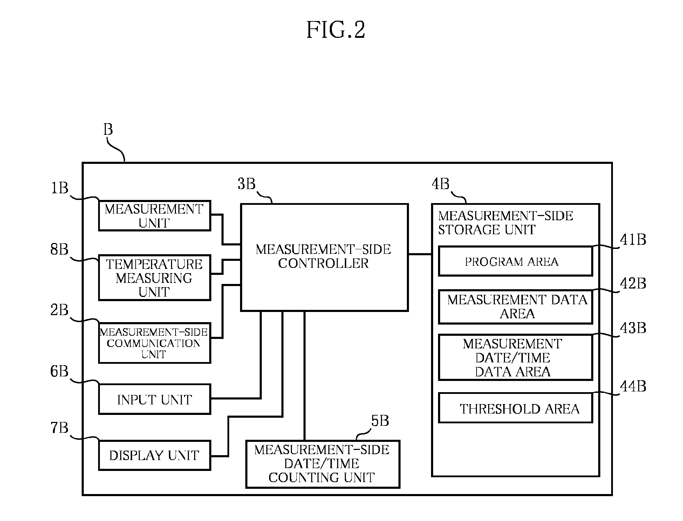

As shown in Fig. 2, the measurement device B includes a measurement unit 1B, a measurement-side communication unit 2B, a measurement-side controller3B,a measurement-sidestorage unit 4B, a measurement-side date/time counting unit 5B, an input unit 6B, a display unit 7B and a temperature measuring unit 8B.

The measurement unit 1B measures a blood glucose level by measuring the glucose concentration in blood. The measurement unit 1B, cooperating with or including a sensor 11B as shown in Fig. 1, includes a receiving portion provided with e. g. terminals for electrical connection to the sensor 11B. The sensor 11B is provided with, for example, a dry reagent and has terminals to be inserted into the receiving portion. In this embodiment, the sensor 11B is a disposable type configured for a single use.

The measurement-side communication unit 2B performs radio communication with the multifunctional portable terminal C. In this embodiment, the measurement-side communication unit 2B is designed to be able to perform communication via Bluetooth (trademark). The specific structure of the measurement-side communication unit 2B is not limitative, and the measurement-side communication unit 2 may comprise a one-chip IC incorporating an antenna and a control element as one unit. The communication standard which the present invention utilizes is not limited to any specific one, and NFC (Near Field Communication) may be employed appropriately.

The measurement-side controller 3B controls the operation and processing of the measurement device B and typically comprises a CPU. Forinstance,the measurement-side controller3B performs control in accordance with a program stored in the measurement-side storage unit 4B.

The measurement-side storage unit 4B stores e.g. a program for realizing the operation or processing of the measurement device B and various kinds of data. The measurement-side storage unit 4B typically comprises a semiconductor memory. In this embodiment, the measurement-side storage unit 4B includes a program area 41B, a measurement data area 42B, a measurement date/time data area 43B and a threshold area 44B.

The program area 41B stores a program for realizing the operation or processing of the measurement device B. The measurement data area 42B stores the measurement data (blood glucose level) obtained by the measurement unit 1B. The measurement date/time data area 43B stores the measurement date/time data obtained by the measurement-side date/time counting unit 5B. The threshold area 44B stores various kinds of thresholds for comparison with measurement data.

The measurement-side date/time counting unit 5B performs the function generally called clock function. For instance, the measurement-side date/time counting unit 5B outputs, as the measurement date/time data, the data on the date and time when measurement is performed by the measurement unit 1B to the measurement-side controller3B. Themeasurement-sidedate/time counting unit 5B may be combined in the measurement-side controller 3B as its one functional part.

The input unit 6B is used by the user to input operations necessary for the operation of the measurement device B. For instance, the input unit 6B comprises push buttons.

The display unit 7B displays the state of the measurement device B or a measured blood glucose level, in accordance with the instruction from the measurement-side controller 3B. The display unit 7B typically comprises a liquid crystal display.

The temperature measuring unit 8B measures the temperature of the measurement device B or the surrounding environment. For instance, the temperature measuring unit 8B comprises a thermocouple, a thermistor or a resistance thermometer.

The multifunctional portable terminal C has a function to communicate with the measurement device B and a voice message output function. Various devices can be used as the multifunctional portable terminal C, but typically a mobile phone is used. Examples of a mobile phone that can be used as the multifunctional portable terminal C include what is called a smart phone powered by an operating system such as Android OS (trademark) or iOS (trademark). Terminals called a tablet or a tablet computer, which do not have a telephone function, may be used as the multifunctional portable terminal C.

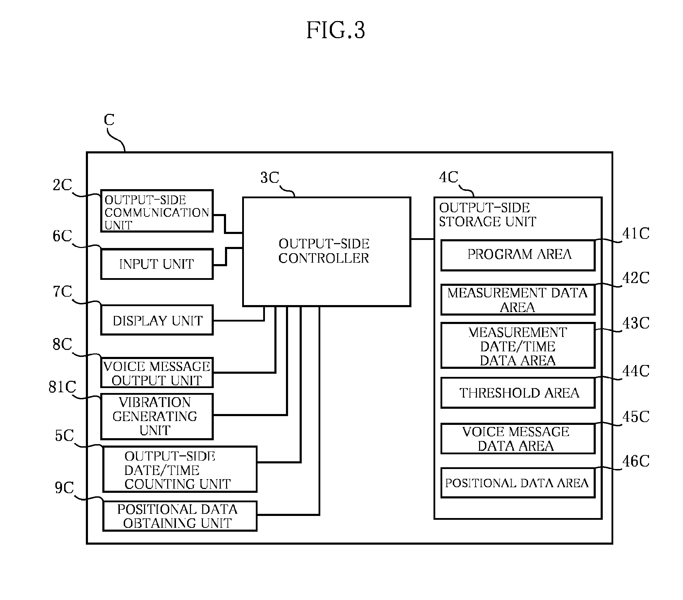

As shown in Fig. 3, the multifunctional portable terminal C includes an output-side communication unit 2C, an output-side controller 3C, an output-side storage unit 4C, an output-side date/time counting unit 5C, an input unit 6C, a display unit 7C, a voice message output unit 8C, a vibration generating unit 81C and a positional data obtaining unit 9C.

The output-side communication unit 2C performs radio communication with the measurement device B. In this embodiment, the output-side communication unit 2C is designed to be able to perform communication via Bluetooth (trademark). The specific structure of the output-side communication unit 2C is not limitative,andtheoutput-side communication unit2C may comprise a one-chip IC incorporating an antenna and a control element as one unit. As noted before, the communication standard which the present invention utilizes is not limited to any specific one, andNFC (Near Field Communication) may be employed appropriately.

In this embodiment, the output-side communication unit 2C is designed to be able to perform radio communication based on IMT-2000 (International Mobile Telecommunication 2000) developed by the International Telecommunication Union. The multifunctional portable terminal C is accessible to the Internet (public network) illustrated in Fig. 1 by radio based on IMT-2000. The output-side communication unit 2C may comprise two separately provided IC chips one of which performs radio communication based on Bluetooth (trademark) and the other of which performs radio communication based on IMT-2000. Alternatively, the output-side communication unit 2C may comprise a single-chip IC that correctively performs both types of wireless communication.

The output-side controller 3C controls the operation and processing of the multifunctional portable terminal C and typically comprises a CPU. For instance, the output-side controller 3C performs control in accordance with a program stored in the output-side storage unit 4C.

The output-side storage unit 4C stores a program for realizing the operation or processing of the multifunctional portable terminal C and various kinds of data. The output-side storage unit 4C typically comprises a semiconductor memory. When the multifunctional portable terminal C is a smart phone, the output-side storage unit 4C may comprise a combination of a ROM, an SD memory card and so on. In this embodiment, the output-side storage unit 4C includes a program area 41C, a measurement data area 42C, a measurement date/time data area 43C, a threshold area 44C, a voice message data area 45C and a positional data area 46C.

The program area 41B stores programs for realizing the operation or processing of the multifunctional portable terminal C. When the multifunctional portable terminal C is a smart phone, the programs include an operating system such as Android OS (trademark) or iOS (trademark) and various application programs. The application programs correspond to application software widely known as "app", which are typically delivered to the multifunctional portable terminal C via the Internet (public network) (see Fig. 1) . The measurement data area 42C stores the measurement data (blood glucose level) transmitted from the measurement device B. The measurement date/time data area 43C stores the measurement date/time data transmitted from the measurement device B. The threshold area 44C stores various kinds of thresholds for comparison with measurement data.

The voice message data area 45C stores various kinds of voice message data to be outputted from the voice message output unit 8C. The positional data area 46C stores the positional data obtained by the positional data obtaining unit 9C and also stores data on a position to be compared with the positional data.

The output-side date/time counting unit 5C performs the function generally called clock function and outputs the current time to the output-side controller 3C. The output-side date/time counting unit 5C may be combined in the output-side controller 3C as its one functional part. The output-side controller 3C may correct the time of the output-side date/time counting unit 5C by using the time data from the Internet.

The input unit 6C is used by the user to input operations. Typically, the input unit 6C comprises a transmissive touch screen laid on the display unit 7C. Examples of the touch screen include a capacitive touch screen and a resistive touch screen. The input unit 6C may include push buttons in addition to the touch screen, as required.

The display unit 7C displays various kinds of information as letters or patterns in accordance with instructions from the output-side controller 3C. Typically, the display unit 7C comprises a liquid crystal display or an organic EL display. When the multifunctional portable terminal C is a smart phone, the display unit 7C can show full-color images.

The voice message output unit 8C outputs voice messages based on voice message data, in accordance with instructions from the output-side controller 3C. For instance, the voice message output unit 8C comprises an amplifier and a speaker. The voice message output unit 8C may further include an earphone terminal and an earphone to be connected to the earphone terminal. The voice message output unit 8C is not limited to the type that transmits voice messages to the user's ears by vibrating air. For instance, the voice message output unit 8C may be of the bone conduction type. According to the voice message output unit 8C of the bone conduction type, voice messages are conducted through the bones of the jaw or the skull to the inner ear and to the brain. This type of voice message output unit 8C is designed to directly apply vibration to the bone called mastoid process located behind the user's ear.

The vibration generating unit 81C includes a vibration source for vibrating the entirety of the multifunctional portable terminal C and incorporates e.g. a small-sized motor. Due to the vibration generated by the vibration generating unit 81C, the user carrying the multifunctional portable terminal C notices that some kind of information is given. In the operation control of the measurement system A described below, in addition to the display on the display unit 7C and voice message output by the voice message output unit 8C, vibration generated by the vibration generating unit 81C is used as auxiliary information notifying means. The judgment on whether to cause the vibration generating unit 81C to generate vibration or not may be made in accordance with whether or not the multifunctional portable terminal C is set to a so-called manner mode.

The positional data obtaining unit 9C functions to find out the current position of the multifunctional portable terminal C. For instance, the positional data obtaining unit 9C performs Global Positioning System (hereinafter referred to as GPS) measurement. When the multifunctional portable terminal C performs radio communication based on the above-described IMT-2000 standard, the current position may be found out based on what is called base station information. When the multifunctional portable terminal C performs radio communication based on wireless LAN standard, the current position may be found out based on the access point information.

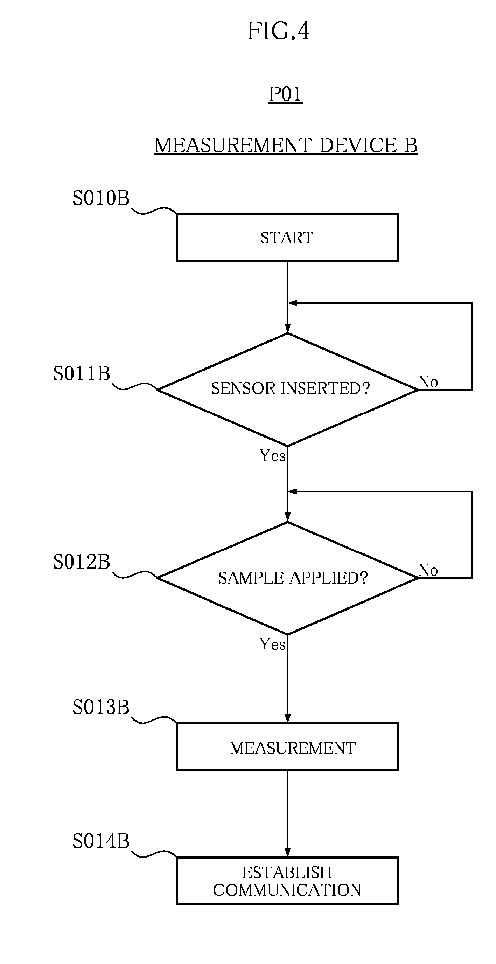

The operation control of the measurement system A is described below with reference to Figs. 4-14. Although the measurement device B is described below as a device that is capable of performing all of the processes P01-P11 illustrated in Figs. 4-14, the measurement device B may be configured to perform only selected ones of these processes or perform other processes in addition to these processes. In the measurement system A, which of the processes P01-P11 is to be performed can be selected appropriately by the input operation in the measurement device B or the multifunctional portable terminal C. Alternatively, the above-described app in the multifunctional portable terminal C may automatically select the process P01-P11 in accordance with a certain algorism.

### <Process P01>

Fig. 4 is a flow chart illustrating the process P01. In the process P01, the steps from the measurement by the measurement device B to the establishment of communication are performed. First, the measurement device B is made ready for operation (Step S010B). Specifically, for instance, the measurement device B is brought into a standby state with a charged battery mounted in it. Then, the sensor 11B is inserted, whereby the measurement device B, which has been in the standby state, is powered on (Step S011B).

Then, the user applies a certain amount of blood to an end of the sensor 11B, and the application of blood is detected e.g. electrically (Step S012B). The measurement unit 1B measures the blood glucose level in the blood (Step S013B). When the blood glucose level measurement by the measurement unit 1B is completed, the measurement result is transmitted to the measurement-side controller 3B as the measurement datum and stored in the measurement data area 42B of the measurement-side storage unit 4B.

In the measurement device B, when the measurement step (Step S013B) is completed, communication with the multifunctional portable terminal C is automatically established (Step S014B). For instance, in the case of radio communication based on Bluetooth (trademark) standard, the pairing process as the initial setting process, in which the measurement device B and the multifunctional portable terminal C are caused to recognize each other, has been completed before the process P01. Thus, in Step S014B, communication is established when the multifunctional portable terminal C responds to the request for connection from the measurement device B. In this way, measurement by the measurement device B is completed, and the system is ready to transmit the measurement result to the multifunctional portable terminal C. In the processes described below, unless otherwise mentioned, the same process as described above is performed in the measurement device B even if different reference signs are applied to the corresponding steps for convenience.

### <Process P02>

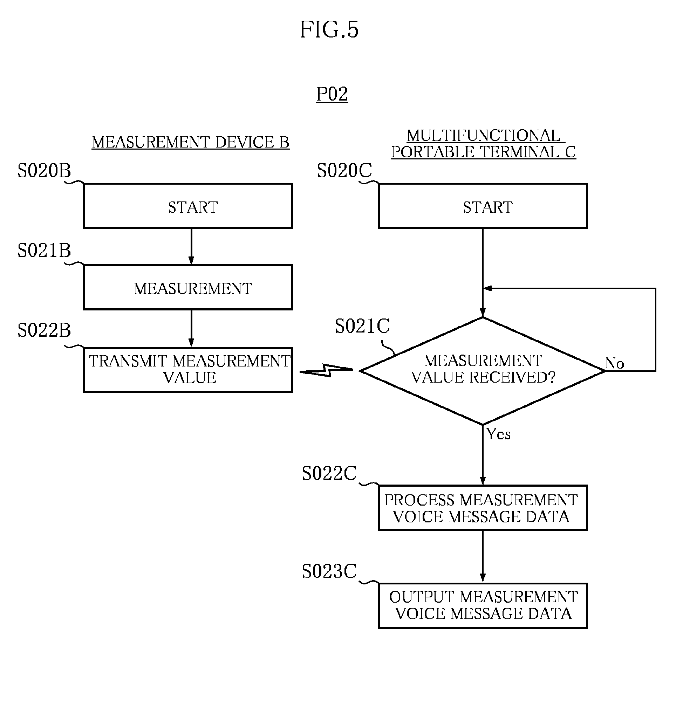

Fig. 5 is a flow chart illustrating the process P02. In the process P02, the blood glucose level measured by the measurement device B is outputted as a voice message (is read out) by the multifunctional portable terminal C. Step S020B and Step S021B in the measurement device B are the same as the above-described Step S010B and Step S013B, respectively. Thus, before Step S022B, the steps corresponding to Steps S010B-Step S014B have been completed. In this state, in response to an instruction from the measurement-side controller 3B, the measurement datum stored in the measurement data area 42B of the measurement-side storage unit 4B is transmitted from the measurement-side communication unit 2B (Step S022B).

The multifunctional portable terminal C is set in advance to a standby state or a power-on state (Step S020C). In order for the multifunctional portable terminal C to perform the process described below, a predetermined program (app) needs to be activated in the multifunctional portable terminal C. The activation of the app may be performed by the user's operation using the input unit 6C or may be performed automatically when the multifunctional portable terminal C is powered on. Once the app is activated in the multifunctional portable terminal C, the app remains activated in the background even after the multifunctional portable terminal C is turned to a standby state.

The app is stored in the program area 41C of the output-side storage unit 4C in advance. The app may be downloaded from the Internet by accessing from the multifunctional portable terminal C to a predetermined website via a URL (or a QR code) attached to the measurement device B after the user purchased the multifunctional portable terminal C.

Upon receiving a reception request from the measurement device B, the output-side controller 3C receives the measurement datum by the output-side communication unit 2C as a process predetermined in the app and stores the measurement datum in the measurement data area 42C of the output-side storage unit 4C (Step S021C: Yes). Until reception of the measurement data from the measurement device B is completed, the multifunctional portable terminal C maintains the standby state (Step S021C: No).

Then, the output-side controller 3C selects or generates a measurement voice message datum. Specifically, the output-side controller 3C refers to the measurement data area 42C of the output-side storage unit 4C for the measurement datum. This measurement datum represents the measuredblood glucose level in blood. Then, the output-side controller 3C converts the blood glucose level to a numerical value expressed in a predetermined unit. Alternatively, a numerical value expressed in a predetermined unit may be generated in the measurement device B and the measurement datum including the numerical value may be transmitted. The voice message data area 45C of the output-side storage unit 4C stores a plurality of voice message data corresponding to various numerical values. From these voice message data stored in the voice message data area 45C, the output-side controller 3C selects the voice message datum corresponding to the measurement datum as the measurement voice message datum. Alternatively, the output-side controller 3C may generate the measurement voice message datum corresponding to the numerical value, in accordance with the algorism contained in the app. Alternatively, the measurement voice message datum may be generated by selecting partial voice message data stored in the voice message data area 45C of the output-side storage unit 4C and combining these data together (Step S022C).

Then, the output-side controller 3C causes the voice message output unit 8C to output the measurement voice message datum (S023C). Thus, the user catches the measurement value read out by the multifunctional portable terminal C and hence learns the measurement result. As illustrated in Fig. 1, the blood glucose level as the measurement value may be displayed on the display unit 7B of the measurement device B or the display unit 7C of the multifunctional portable terminal C.

### <Process P03>

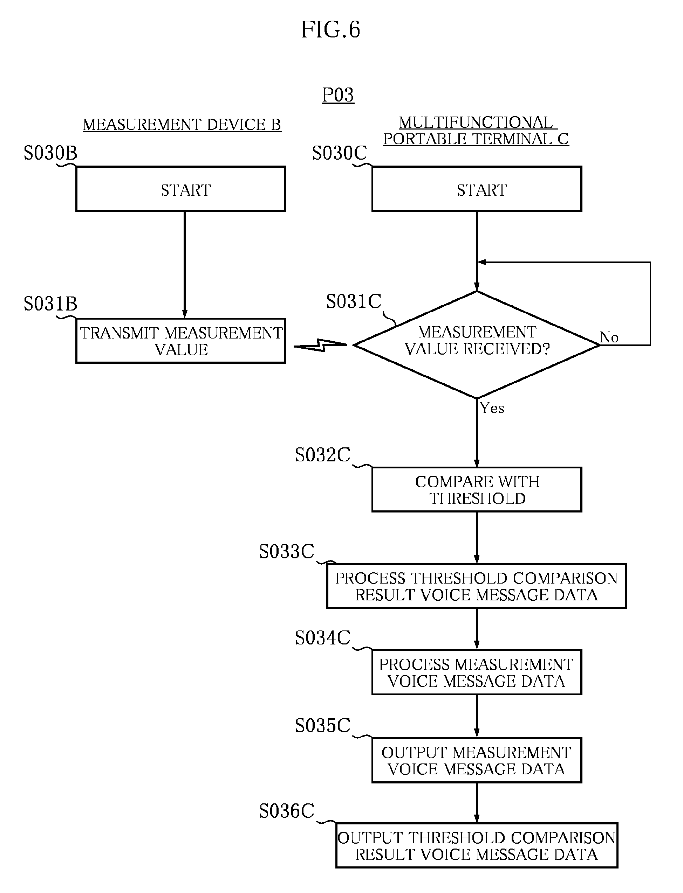

Fig. 6 is a flow chart illustrating the process P03. In the process P03, in addition to the measurement value voice message, a voice message indicating the result of comparison with a threshold is out putted from the multifunctional portable terminal C. First, Step S030B and Step S031B are performed in the measurement device B. The completion of Step S031B corresponds to completion of the above-described Steps S010B-S014B.

In the multifunctional portable terminal C, Step S030C and Step S031C are first performed. These Steps S030C and S031C are the same as Step S020C and S021C, respectively.

Then, the output-side controller 3C performs a threshold comparing process (Step S032C). The threshold area 44C of the output-side storage unit 4C stores various threshold data. In Step S032C, the measurement value from the measurement device B is compared with the threshold data. Examples of the thresholds include a blood glucose level that is considered to be high or low for a healthy person, a diabetes patient or an individual who uses the measurement system A. These thresholds may be set in advance by the installation of the app or may be appropriately set by the input operation by the user after the app is activated.

Then, in accordance with the result of the threshold comparing process, the output-side controller 3C performs threshold comparison result voice message data processing (Step S033C). The voice message data area 45C of the output-side storage unit 4C stores a plurality of threshold comparison result voice message data corresponding to various threshold comparison results. For instance, when the measurement value is higher than a threshold, the threshold comparison result voice message that says "It's high" may be selected. When the measurement value is lower than a threshold, the threshold comparison result voice message that says "It's low" or "It's low. Please measure again" may be selected.

Then, Step S034C is performed. Step S034C is the same as Step S022C. Then, Step S035C and Step S036C are performed successively. Step S035C is the same as Step S023C, and the measurement voice message datum is outputted in this step. In Step S036C, the threshold comparison result voice message data selected in Step S033C is outputted from the voice message output unit 8C.

### <Process P04>

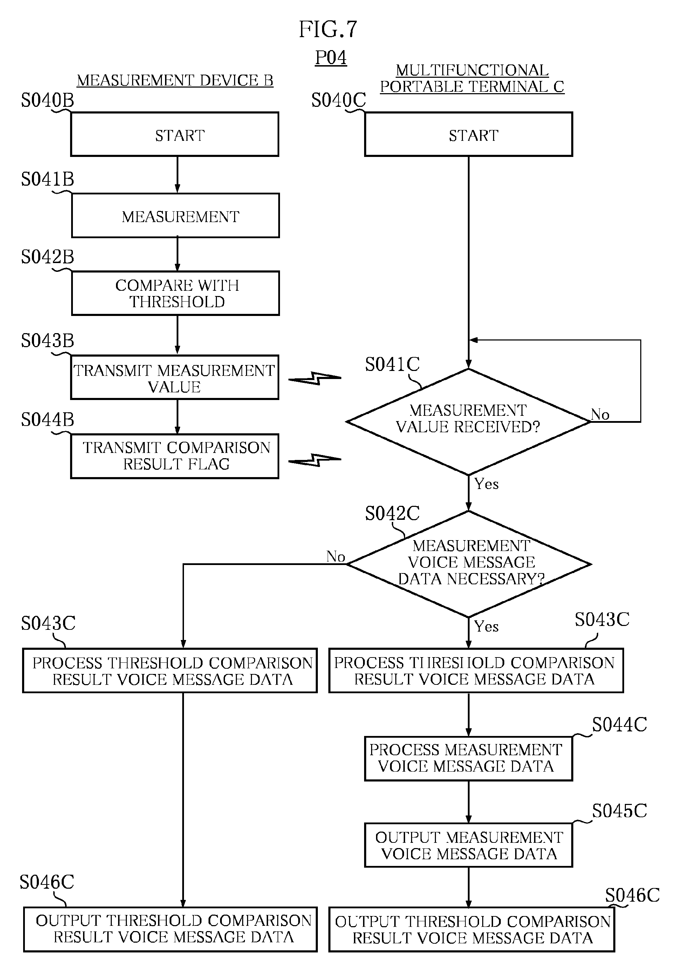

Fig. 7 is a flow chart illustrating the process P04. In the process P04, the measurement device B transmits a comparison result flag along with the measurement datum. First, Step S040B and S041B are performed in the measurement device B. Then, the measurement-side controller 3B performs the threshold comparing process (Step S042B). The threshold area 44B of the measurement-side storage unit 4B stores a plurality of threshold data for comparison with the measurement value. The measurement value from the measurement device B is compared with the threshold data. These threshold data may be the same as the threshold data used in the above-described process P03. Alternatively, the upper and lower limits of the range of blood glucose levels that can be properly measured by the measurement device B may be set as the thresholds. The measurement-side controller 3B compares the measurement value with the thresholds and generates a comparison result flag in accordance with the result of comparison. The comparison result flag may be a flag indicating that the measurement value is relatively high or low or a flag indicating that the measurement value is higher than the upper limit or lower than the lower limit of the properly measurable blood glucose level range. These thresholds may be set in advance by the installation of the app or may be appropriately set by the input operation by the user after the app is activated.

Then, the measurement-side controller 3B successively performs Step S043B and Step S044B. Step S043B is the same as Step S022B. In Step S044B, the measurement-side controller 3B causes the measurement-side communication unit 2B to transmit the comparison result flag generated in Step S042B.

The multifunctional portable terminal C receives the measurement datum and the comparison result flag by performing Step S040C and then Step S041C. Then, in Step S042C, the comparison result flag is compared with a table or list prepared in advance, whereby it is determined whether or not the generation or selection of a measurement voice message data is necessary, i.e., whether or not reading out of the measurement value is necessary. Specifically, when the comparison result flag indicates that the measurement value is relatively high or low (Step S042C: Yes), Steps S043C-S046C are performed. Steps S043C-S046C are the same as Step S033C-S036C.

When the comparison result flag indicates that the measurement value is higher than the upper limit or lower than the lower limit of the measurable blood glucose level range (Step S042C: No), the output-side controller 3C selects the threshold comparison result voice message datum corresponding to the comparison result flag from the data stored in the voice message data area 45C of the output-side storage unit 4C (Step S043C). These threshold comparison result voice message data may correspond to the voice messages such as: "the measurement value is not within the measurable range", "please measure again", "the measurement value did not fall within the measurable range for the second time in a row" or "the measurement device may not be operating properly". Then, the output-side controller 3C causes the voice message output unit 8C to output the selected threshold comparison result voice message datum (Step S046C). Inthiscase, since the measurement value is not within the measurable range, the steps for reading out the measurement value are not performed.

### <Process P05>

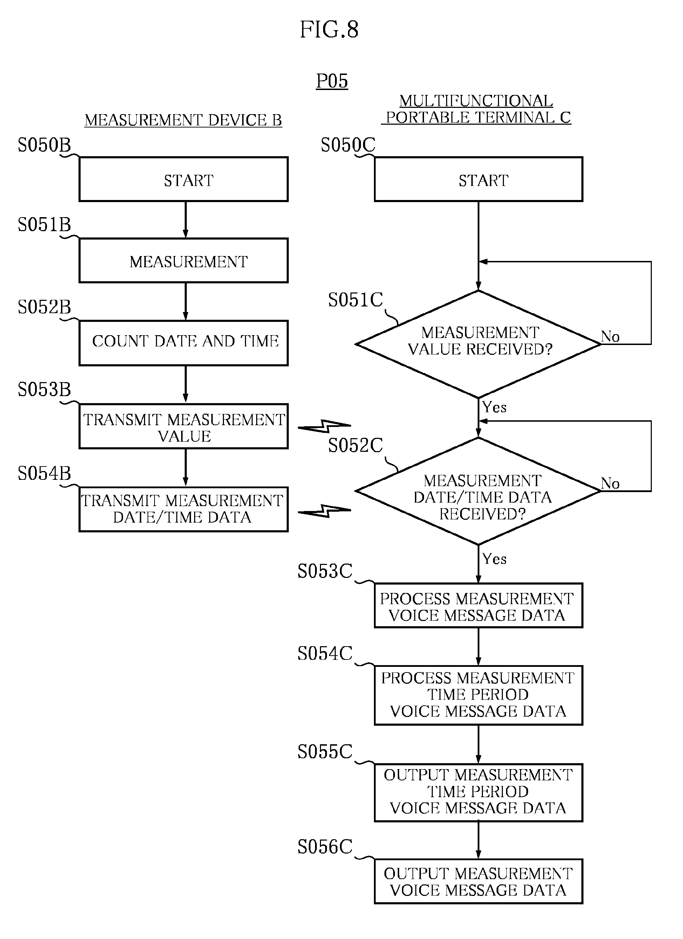

Fig. 8 is a flow chart illustrating the process P05. In the process P05, the process related to the date and time when the measurement is made by the measurement device B is performed. First, Steps S050B and S051B are performed in the measurement device B. Also, the measurement-side controller 3B obtains from the measurement-side date/time counting unit 5B the datum on the date and time when Step S051B is performed and stores the datum in the measurement date/time data area 43B of the measurement-side storage unit 4B (Step S052B). Then, the measurement-side controller 3B causes the measurement-side communication unit 2B to perform transmission of the measurement datum (Step S053B) and transmission of the measurement date/time datum (Step S054B) .

In the multifunctional portable terminal C, after the Step S050C, the output-side controller 3C receives the measurement datum (Step S051C) and the measurement date/time datum (Step S052C). Then, the output-side controller 3C selects or generates the measurement voice message datum corresponding to the measurement datum (Step S053C). The voice message data area 45C of the output-side storage unit 4C stores a plurality of measurement time period voice message data. For instance, these measurement time period voice message data correspond to voice messages such as "before breakfast", "after breakfast", "before lunch", "before supper" or "after supper". The output-side controller 3C selects the measurement time period voice message datum corresponding to the received measurement date/time datum (Step S054C). Then, the output-side controller 3C outputs the measurement time period voice message datum (Step S055C) and then the measurement voice message datum (Step S056C) from the voice message output unit 8C. Specifically, when the measurement date/time datum is received, the output-side controller 3C determines the measurement time period in which the measurement date/time falls in by referring to a table, a list or thresholds which relate measurement date/time data to measurement time periods. The table, list or thresholds are stored in the output-side storage unit 4C. The table, list or thresholds may be set in advance by the installation of the app or may be appropriately set by the input operation by the user after the app is activated.

As a variation of the process P05, a measurement time period flag may be generated in the measurement device B and transmitted in the measurement date/time data transmission step (Step S054B) to the multifunctional portable terminal C together with or instead of the measurement date/time datum. Based on the received measurement time period flag, the multifunctional portable terminal C performs selection of the appropriate measurement time period voice message datum (Step S053C). The measurement time period flags are prepared correspondingly to the time periods such as "before breakfast" "after lunch" or "before supper". Typically, generation of a measurement time period flag in the measurement device B is performed, before or after the measurement step (Step S051B), by the user's inputting operation of the current time period by using the input unit 6B of the measurement device B. Alternatively, the measurement-side controller 3B may generate a measurement time period flag based on the measurement date/time datum by referring to a predetermined table, list or thresholds.

As another variation of the process P05, voice message data for the reading of the measurement date/time itself may be stored in the voice message data area 45C of the output-side storage unit 4C. Alternatively, voice message data for the measurement date/time itself may be generated by the output-side controller 3C. In this case, there processes are performed in a step corresponding to Step S054C, and in Step S056C, measurement date/time itself is outputted as a voice message from the voice message output unit 8C.

### <Process P06>

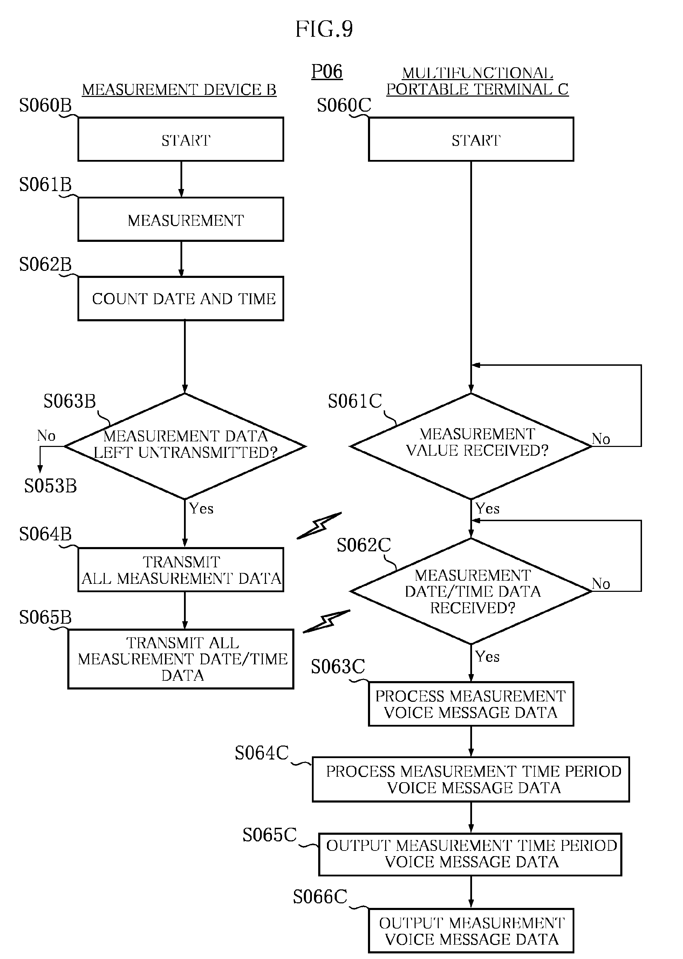

Fig. 9 is a flow chart illustrating the process P06. The process P06 is similar to the process P05 but differs from the process P05 in that a plurality of data are collectivelyprocessed. After the Steps S060B-S062B are performed, it is determined whether or not untransmitted measurement data are left in the measurement data area 42B of the measurement-side storage unit 4B (Step S063B). For instance, such determination may be made possible by applying a transmission completion flag to each of the measurement data which have already been transmitted. For instance, measurement data are left untransmitted if measurement (StepS051B, S052B) is performed a plurality of times in the process P05 but the transmission steps (Steps S053B, S054B) are not properly performed and hence the process P05 is not completed because of such a reason as multifunctional portable terminal C is not carried or communication is not properly established. In such a case, a plurality of measurement date/time data corresponding to the untransmitted measurement data are stored in the measurement date/time data area 43B of the measurement-side storage unit 4B. In the process P06, when untransmitted measurement data are found (Step S063B: Yes), the untransmitted measurement data and the corresponding measurementdate/time data are collectively transmitted in Steps S064B and S065B, along with the measurement datum and measurement date/time datum newly obtained in Steps S061B and S062B. When untransmitted measurement data are not found (Step S063B: No), the same process as the process P05 is performed.

The multifunctional portable terminal C collectively receives these measurement data and measurement date/time data by performing Steps S061C and S062C. Steps S063C-S066C may be performed collectively with respect to all of these measurement data and measurement date/time data. Alternatively, the latest measurement datum may be selected from the plurality of measurement data, and Steps S063C-S066C may be performed only with respect to the latest measurement datum and the corresponding measurement date/time datum.

### <Process P07>

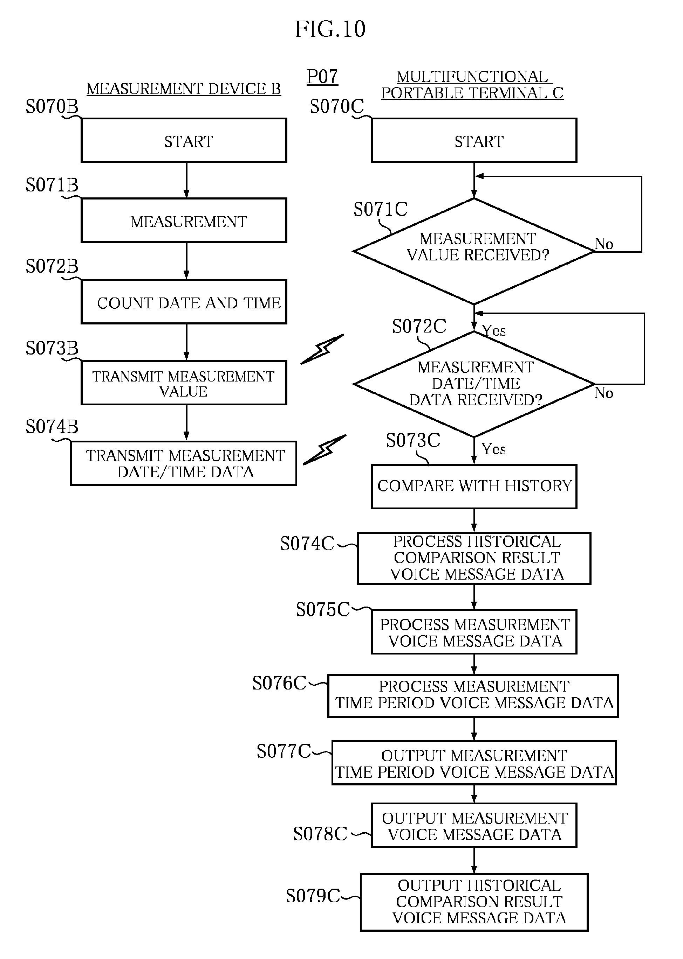

Fig. 10 is a flow chart illustrating the process P07. In the process P07, the results of comparison with the history of measurement performed in the past are outputted. In the measurement device B, Steps S070B-S074B are performed. In the multifunctional portable terminal C, Steps S070C-S072C are performed, whereby the measurement datum and measurement date/time datum are received. Based on the received measurement date/time datum, the output-side controller 3C determines e.g. the measurement time period. The output-side controller 3C then picks up a measurement datum on measurement performed in the same measurement time period, from the measurement data already stored in the measurement data area 42C of the output-side storage unit 4C. The output-side controller 3C then compares the newly obtained measurement datum with the picked-up measurement datum (Step S073C). The voice message data area 45C of the output-side storage unit 4C stores a plurality of historical comparison result voice message data corresponding to the results of historical comparison. For instance, the historical comparison result voice message data correspond to the voice messages such as: "higher than the last measurement value in the same measurement period", "lower than the last measurement value in the same measurement period", "the blood glucose level is now 120, dropped by 10 from the last value 130 before breakfast". From these historical comparison result voice message data, the output-side controller 3C selects the historical comparison result voice message datum corresponding to the result of the historical comparison process of Step S073C (Step S074C). Then, Steps S075C-S078C are performed, whereby the measurement voice message datum and the measurement time period voice message datum are outputted from the voice message output unit 8C. Then, the historical comparison result voice message datum is outputted from the voice message output unit 8C (Step S079C).

### <Process P08>

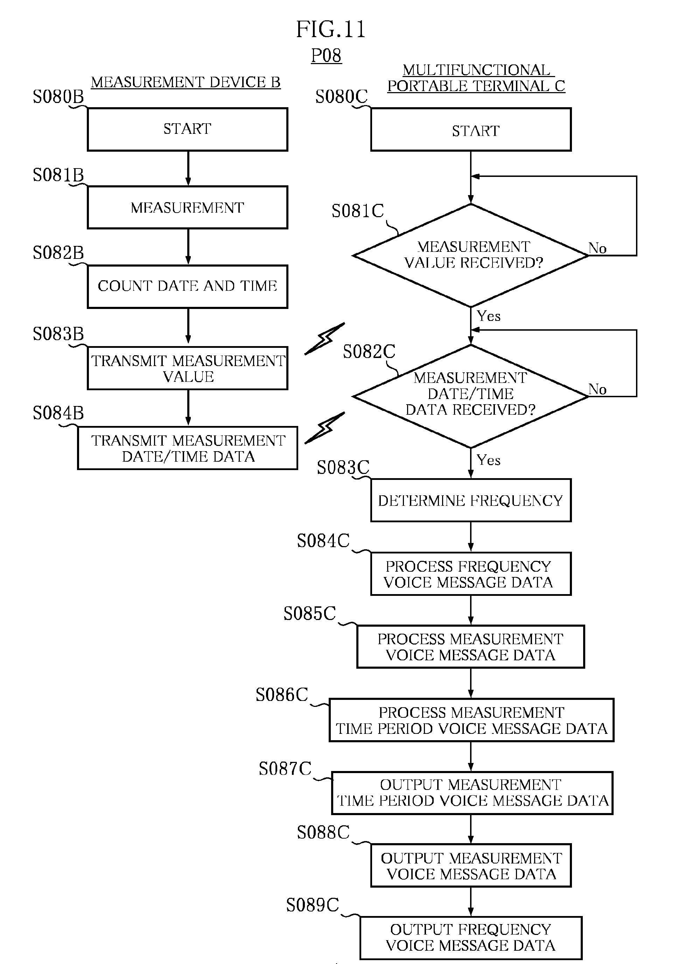

Fig. 11 is a flow chart illustrating the process P08. The process P08 includes the steps of checking whether or not the frequency of measurement by the user is proper and outputting the check result. First, in the measurement device B, Steps S080B-S084B are performed. In the multifunctional portable terminal C, Steps S080C-S082C are performed, whereby the measurement datum and measurement date/time datum are received. Then, the output-side controller 3C picks up, from the measurement date/time data area 43C of the output-side storage unit 4C, the measurement date/time datum corresponding to the latest one of the measurement data in the past stored in the measurement data area 42C, and then compares the picked-up measurement date/time datum with the received measurement date/time datum (Step S083C) . In this step, the measurement date/time datum in the same measurement time period as the received measurement date/time datum may be picked up. The voice message data area 45C of the output-side storage unit 4C stores frequency voice message data. The frequency voice message data may correspond to voice messages such as: "Measurement is performed every day", "X days have passed since the last measurement", "It has been a long time since the last measurement". Based on the result in Step S083C, the output-side controller 3C selects the appropriate frequency voice message datum (Step S084C). Then, Steps S085C- S088C are performed, whereby the measurement voice message datum and the measurement time period voice message datum are outputted from the voice message output unit 8C. Then, the frequency voice message datum is outputted from the voice message output unit 8C (Step S089C).

### <Process P09>

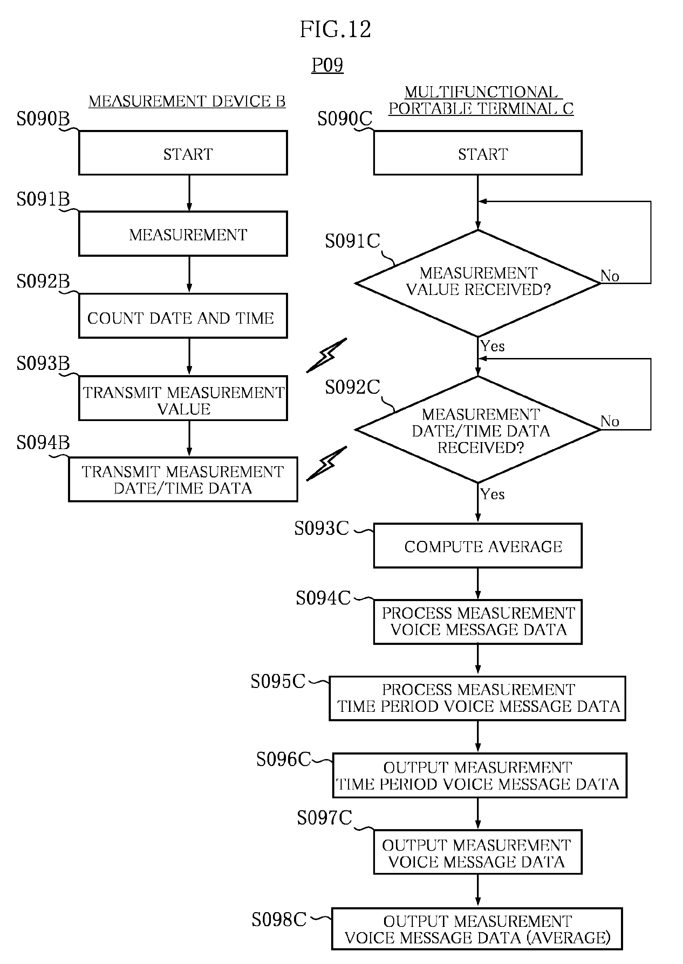

Fig. 12 is a flow chart illustrating the process P09. In the process P09, the average of the measurement data in the past is outputted. First, in the measurement device B, Steps S090B-S094B are performed. In the multifunctional portable terminal C, Steps S090C-S092C are performed, whereby the measurement datum and the measurement date/time datum are received. The output-side controller 3C, picks up, from a plurality of measurement data in the past stored in the measurement data area 42C of the output-side storage unit 4C, a plurality of measurement data of the same measurement time period as that of the received measurement datum and computes the average of these picked-up data. (Step S093C). The output-side controller 3C generates a voice message datum corresponding to the average by the process similar to the generation of the measurement voice message datum. For instance, the voice message datum corresponds to the voice message saying "the average of this time period is X". Then, the output-side controller 3C performs Steps S094C-S097C, whereby the measurement time period voice message datum and the measurement voice message datum are outputted from the voice message output unit 8C. Then, the measurement voice message datum corresponding to the average is outputted from the voice message output unit 8C (Step S098C).

As a variation of the process P09, in addition to the average computation process (Step S093C), the newly received measurement datum may be compared with the average and the difference of the measurement datum from the average maybe computed. In this case, in the measurement voice message data processing (Step S094C), a voice message datum for a voice message such as "higher by 10 than the average" may be generated. In the step of outputting the measurement voice message datum (Step S098C), the result of comparison with the average is outputted along with the measurement value from the voice message output unit 8C.

### <Process P10>

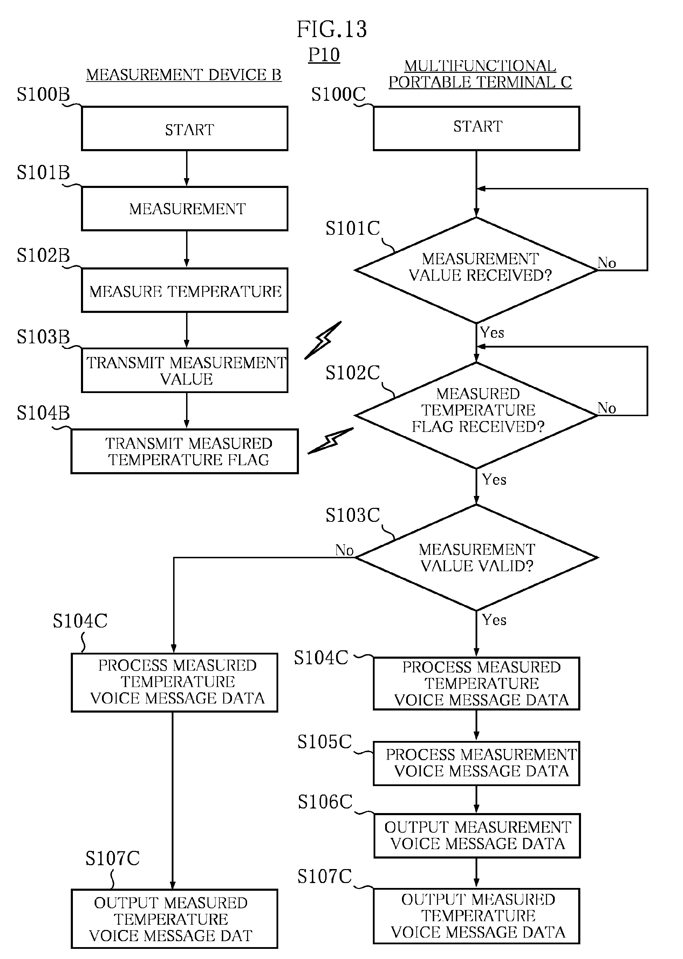

Fig. 13 is a flow chart illustrating the process P10. The process P10 includes a step related to the temperature of the measurement device B. In the measurement device B, Steps S100B and S101B are performed. Then, the measurement-side controller 3B obtains the measured temperature from the temperature measuring unit 8B and generates a measured temperature flag based on the measured temperature (Step S102B). The measured temperature flag corresponds to whether or not the temperature of the measurement device B is within an allowable range suitable for measurement. Alternatively, the measured temperature flag may correspond to whether the temperature of the measurement device B is relatively high or relatively low. Then, the measurement-side controller 3B performs Steps S103B and 104B, whereby the measurement datum and the measured temperature flag are transmitted.

In the multifunctional portable terminal C, Steps S100C-S102C are performed, whereby the measurement datum and the measured temperature flag are received. The voice message data area 45C of the output-side storage unit 4C stores a plurality of measured temperature voice message data corresponding to measured temperature flags. Based on the measured temperature flag, the output-side controller 3C determines whether or not the measurement value is valid (Step S103C). For instance, when the measured temperature flag indicates that the temperature is within an allowable range but it is relatively high or low (Step S103C: Yes), the output-side controller 3C selects, based on the measured temperature flag, the measured temperature voice message datum corresponding to the voice message saying: "The temperature is relatively high" or "the temperature is relatively low" (Step S104C). Then, the output-side controller 3C performs Steps S105C and S106C, whereby the measurement voice message datum is outputted from the voice message output unit 8C. Then, the measured temperature voice message datum is outputted from the voice message output unit 8C (Step 107C).

On the other hand, when it is found out from the measured temperature flag that the measured temperature is not within the allowable range in Step S103C (Step S103C: No), the output-side controller 3C selects the measured temperature voice message datum corresponding to the voice message saying e.g. "the temperature is abnormal" or "Try measurement again". (Step S104C). This measured temperature voice message datum is outputted from the voice message output unit 8C (Step S107C).

### <Process P11>

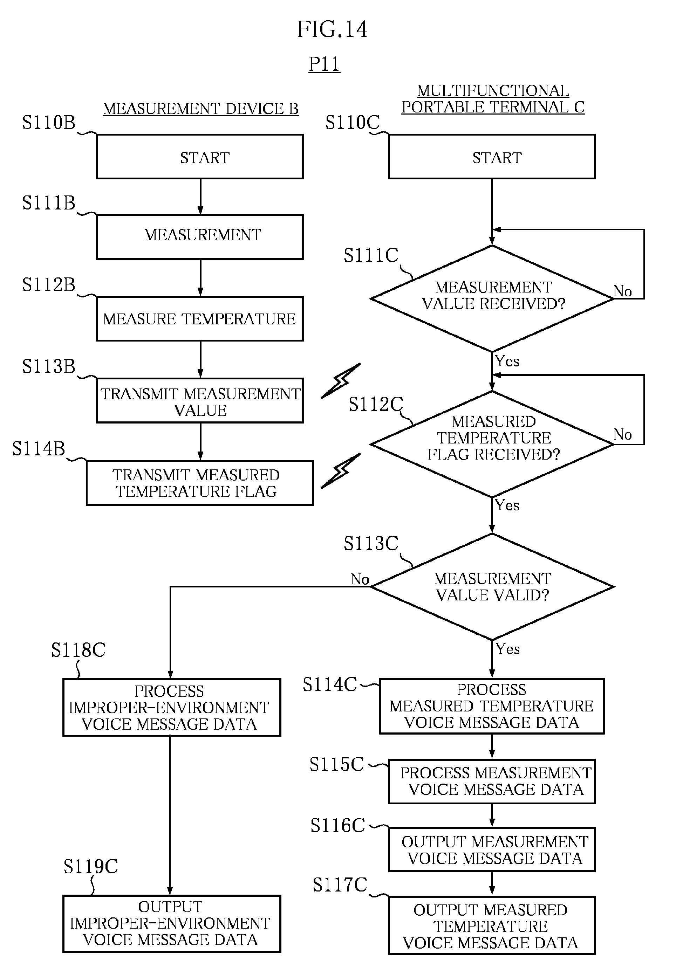

Fig. 14 is a flow chart illustrating the process P11. The process P11 is generally the same as the process P10. However, when the determination is NO in Step S113C, which corresponds to Step S103C of the process P10, the process P11 follows different steps from the steps in the process P10. Specifically, when it is found out from the measured temperature flag that the measured temperature is not within the allowable range in Step S103C (Step S113C: No), the output-side controller 3C checks the flag history to find out whether or not the same or similar measured temperature flag (e.g. indicating that the temperature is not within the allowable range) has been received in the past. When it is found out that the same or similar flag has been received more than a predetermined number of times within a certain period, it is determined that the environment in which the measurement device B is placed is not proper. The voice message data area 45C of the output-side storage unit 4C stores improper-environment voice message data, one of which may correspond to a voice message saying "be careful where you put the measurement device". The output-side controller 3C selects the appropriate improper-environment voice message datum (Step S118C). Then, the output-side controller 3C outputs the selected improper-environment voice message datum from the voice message output unit 8C (Step S119C).

The measurement system A may be configured to perform other various processes.

For instance, in the processes P01-P11, the output-side controller 3C of the multifunctional portable terminal C obtains positional information from the positional data obtaining unit 9C. The positional data area 46C of the output-side storage unit 4C stores allowable area data, i.e., data on the area in which voice message output is allowable and inhibited area data, i.e., data on the area in which voice message output should be inhibited. When the obtained positional information indicates a position within the allowable area or a position outside the inhibited area, the output-side controller 3C executes voice message output from the voice message output unit 8C. On the other hand, when the obtained positional information indicates a position outside the allowable area or a position within the inhibited area, the output-side controller 3C executes a process to inhibit the voice message output unit 8C from outputting voice messages. In this case, the fact that the voice message output is inhibited based on the positional information may be notified to the user to for confirmation. This notification may be performed by e.g. displaying a message or a mark on the display unit 7C. Alternatively, in addition to the display on the display unit 7C, vibration may be generated by the vibration generating unit 81C. Moreover, a voice message saying "the data is received, but the blood glucose level is not read out here" may be outputted.

Typically, the positional information is obtained by the positional data obtaining unit 9C of the multifunctional portable terminal C by the execution of a location information app by the output-side controller 3C. Such a location information app is usually prepared as a basic function of the multifunctional portable terminal C, separately from the app for executing the processes P01-P11. The allowable area data and the inhibited area data may be set as desired by the user by the input operation using the input unit 6C of the multifunctional portable terminal C. Specifically, for example, setting the area data includes inputting the location of the center of the allowable or inhibited area or inputting the radius (distance) of a circle defining the allowable or inhibited area. For instance, for the inputting of the center position, a map of a desired area may be displayed on the display unit 7C, and the inputting operationmaybe performed with reference to the map by using the input unit 6C. For instance, the residence of the user may be set as the center of the allowable area. For instance, the location of community facilities may be set as the center of the inhibited area. When the position indicated by the obtained positional information is within the inhibited area, the output-side controller 3C may compute the distance between the position and the center of the inhibited area. The output-side controller 3C may then generate a voice message datum corresponding to the distance and cause the voice message output unit 8C to output a voice message saying e.g. "within the radius Y km area from X (center of the inhibited area) ". It is preferable that the output volume from the voice message output unit 8C is reduced in advance by the output-side controller 3C before this voice message is outputted.

As another example, in the multifunctional portable terminal C, the output-side controller 3C obtains a reference date/time datum from the Internet through the output-side communication unit 2C. Based on the reference date/time datum, the output-side controller 3C corrects the count of the output-side date/time counting unit 5C. Moreover, the output-side controller 3C compares the measurement date/time datum transmitted from the measurement device B and the date/time datum counted by the output-side date/time counting unit 5C. When an error exceeding a certain level is observed in the measurement-side date/time counting unit 5B, a date/time correction datum is outputted from the output-side communication unit 2C to the measurement-side communication unit 2B. Based on the date/time correction datum received by the measurement-side communication unit 2B, the measurement-side controller 3B corrects the count of the measurement-side date/time counting unit 5B. The voice message data area 45C of the output-side storage unit 4C stores a date/time correction voice message datum to indicate that date/time correction has been performed. For instance, the date/time correction voice message datum may correspond to a voice message saying "date/time of the measurement device is corrected". The output-side controller 3C causes the voice message output unit 8C to output the date/time correction voice message datum.

The advantages of the measurement system A are described below.

According to this embodiment, the measurement device B needs to have only the function to measure blood glucose levels and the function to transmit the measurement values, and the generation of voice message data related to the measurement values is per formed by the multifunctional portable terminal C. Various kinds of voice messages exemplarily described in the process P02-P11 can be outputted by improving the app to be executed by the multifunctional portable terminal C, so that the measurement device B does not need to have a complicated structure. In particular, when the multifunctional portable terminal C is a smart phone, the hardware cost can be reduced by large distribution. The app can be developed at a relatively low cost. Since the number of users is expected to increase, the app maybe distributed for free or can be sold at a considerably low price. In this way, the measurement system A is provided which is capable of outputting various kinds of voice messages while having a simple structure.

Since the output-side communication unit 2C is connectable to the Internet, the app to be executed by the multifunctional portable terminal C can be updated easily and various voice message data can be added easily. In particular, when the user makes setting to allow automatic update, the update of the app or addition of various voice message data is performed at a proper timing, without requiring the user to care about it.

According to the process P01, the user can quickly perform the measurement just by inserting the sensor 11B. Thus, the blood glucose level measurement, which needs to be performed quite often, is performed easily.

According to the process P02, the blood glucose level itself as a measurement value is read out by the multifunctional portable terminal C. It is likely that the user carries the multifunctional portable terminal C, or typically a smart phone, at a position which allows easy catching of voice messages. Thus, when the blood glucose level is read out from the multifunctional portable terminal C, the user properly finds out the blood glucose level without the need for visually checking it.

According to the process P03, whether the blood glucose level is relatively high or relatively low can be found out. In accordance with this, the user can quickly take proper measures such as improving diet or receiving proper medical care.

According to the process P04, the user can find out immediately after performing measurement whether the blood glucose level obtained by the measurement is a valid measurement result or an incorrect measurement result due to some reason. The user's attention can be properly attracted by preparing various kinds of threshold comparison result voice message data.

In the process P05, the measurement time period is read out along with the measurement value. This not only allows the user to find out the measurement time period but also allows the user to form the habit of regularly measuring the blood glucose level.

In the process P06, the latest measurement datum can be selectively read out from a plurality of measurement data. Further, the user can learn the blood glucose level measurements which have not been notified to the user. According to this arrangement, daily measurement results can be effectively utilized even when communication is not always established between the measurement device B and the multifunctional portable terminal C.

In the process P07, the user can find out the result of comparison with a blood glucose level obtained in the same time period in the past. Generally, the tendency of a blood glucose level largely changes between different time periods based on meals. Even when measurement is continuously performed before and after each meal, the user cannot find out the tendency of a blood glucose level in a certain time period if the system is configured to notify only the latest measurement result. According to the process P07, the user can easily and reliably find out the tendency of the blood glucose level in a certain time period.

According to the process P08, the user can find out whether or not the frequency of measurement is proper. Even when the user believes that he or she has been performing the measurement at a proper frequency, the frequency may not be actually sufficient. According to the process P08, such a fact can be notified to the user at a proper timing.

According to the process P09, the user can easily find out how the current measurement value is as compared with the average of the measurement values and, in particular, with the average of the measurement values obtained in the same time period. Such comparison with the average requires continuous storage of measurement results and proper computation. For instance, if the user tries to obtain a comparison result of a measurement value with the average by using the measurement device B only, a considerable amount of work needs to be done, and there is a possibility of making a mistake. According to the process P09, the comparison result with the average can be provided without any burden on the user.

In the process P10, the user can find out whether or not the measured temperature is proper. When the measured temperature is not proper, the user can concentrate on finding out the cause of such an abnormal temperature while omitting the work for checking the blood glucose level. Even when the measured temperature is within the allowable range, by finding out whether the measured temperature is relatively high or low, the relationship with the temperature and the blood glucose level may be found out if necessary, which is helpful.

In the process P11, the user can be notified quickly if the place or manner of storage of the measurement device B or the place where the measurement device B is used is not what the manufacturer of the measurement device B has expected. Thus, the user can quickly restart proper measurement by changing the place or manner of storage or the place of use of the measurement device B. This also contributes prevention of an unexpected failure of the measurement device B.

The measurement system according to the present invention is not limited to the foregoing embodiments. The specific structure of each part of the measurement system according to the present invention may be varied in design in many ways.

The measurement device according to the present invention is not limited to the device that uses a disposable sensor to which a dried reagent is applied. For instance, use may be made of a measurement device that contains an appropriate amount of liquid reagent for a plurality of times of measurement. In this case, the number of measurement operations may be counted by the measurement device, and a flag indicating that the time for exchange of reagent is coming or has come may be transmitted from the measurement device to the multifunctional portable terminal. In this case, a voice message saying e.g. "exchange the reagent" is outputted from the multifunctional portable terminal.

## Claims

1. A measurement system comprising:
a measurement device including a measurement unit for measuring a particular component in a biological sample, a measurement-side communication unit for transmitting by radio a measurement datum obtained by the measurement unit, and a measurement-side controller for controlling the measurement unit and the measurement-side communication unit; and
a multifunctional portable terminal including an output-side communication unit for receiving the measurement datum transmitted from the measurement-side communication unit, an output-side storage unit for storing a plurality of voice message data, an output-side controller for generating a voice message datum or selecting a voice message datum from the voice message data stored in the output-side storage unit based on the measurement datum, and a voice message output unit for outputting the voice message datum generated or selected by the output-side controller.

2. The measurement system according to claim 1, wherein the measurement device includes a measurement-side storage unit for storing a plurality of measurement data obtained by the measurement unit.

3. The measurement system according to claim 2, wherein the output-side controller selects or generates, as the measurement voice message datum, a measurement voice message datum including a numerical value representing the concentration of the particular component expressed in a predetermined unit, and the output-side controller causes the voice message output unit to output the measurement voice message datum.

4. The measurement system according to claim 2 or 3, wherein the output-side storage unit stores a threshold datum to be compared with the measurement datum, and a plurality of threshold comparison result voice message data corresponding to results of comparison between the measurement datum and the threshold datum, and
the output-side controller causes the voice message output unit to output one of the threshold comparison result voice message data which is selected based on a result of comparison between the measurement datum and the threshold datum.

5. The measurement system according to claim 2 or 3, wherein the measurement-side storage unit stores a threshold datum to be compared with the measurement datum,
the measurement-side controller causes the measurement-side communication unit to transmit a comparison result flag generated based on a result of comparison between the measurement datum and the threshold datum,
the output-side storage unit stores a plurality of threshold comparison result voice message data corresponding to a plurality of comparison result flags, and
the output-side controller causes the voice message output unit to output one of the threshold comparison result voice message data which corresponds to the comparison result flag transmitted from the measurement-side communication unit.

6. The measurement system according to any one of claims 2-5, wherein the measurement device includes a measurement-side date/time counting unit for counting date and time,
the measurement-side controller obtains from the measurement-side date/time counting unit date and time at which the measurement datum is obtained to generate a measurement date/time datum, and
the measurement date/time datum is stored in the measurement-side storage unit.

7. The measurement system according to claim 6, wherein the multifunctional portable terminal includes an output-side date/time counting unit for counting date and time,
the output-side communication unit is capable of performing communication using a public network,
the output-side controller corrects a count of the output-side date/time counting unitbasedon a reference date/time datum which the output-side communication unit obtains via the public network,
the output-side controller compares the measurement date/time datum transmitted from the measurement device with a date/time datum counted by the output-side date counting unit and causes the output-side communication unit to transmit a date/time correction datum when an error exceeding a predetermined level is observed in the measurement-side date/time counting unit, and
the measurement-side controller corrects a count of the measurement-side date/time counting unit based on the date/time correction datum received by the measurement-side communication unit.

8. The measurement system according to claim 6 or 7, wherein the measurement-side controller transmits a plurality of measurement data and a plurality of measurement date/time data corresponding to the measurement data, and
the output-side controller generates or selects a measurement voice message datum corresponding to latest one of the plurality of measurement data received by the output-side communication unit and causes the voice message output unit to output the measurement voice message datum.

9. The measurement system according to any one of claims 6-8, wherein the output-side storage unit stores a plurality of measurement time period voice message data corresponding to a plurality of measurement date/time data, and
the output-side controller selects one of the measurement time period voice message data which corresponds to the measurement date/time datum and causes the voice message output unit to output the measurement date/time datum.

10. The measurement system according to any one of claims 6-9, wherein the output-side storage unit stores a plurality of historical comparison result voice message data corresponding to results of comparison between a measurement datum and a plurality of measurement data obtained in the past, and
the output-side controller causes the voice message output unit to output one of the historical comparison result voice message data which corresponds to a result of comparison between a measurement datum received by the output-side communication unit and the plurality of measurement data obtained in the past and stored in the output-side storage unit.

11. The measurement system according to any one of claims 6-10, wherein the output-side storage unit stores a plurality of frequency voice message data corresponding to frequencies of measurement, and
the output-side controller selects one of the frequency voice message data based on a result of comparison between a measurement date/time datum related to a newly obtained measurement datum and latest one of the measurement date/time data stored in the output-side storage unit, and causes the voice message output unit to output the frequency voice message data.

12. The measurement system according to any one of claims 6-11, wherein the output-side controller computes an average of a plurality of measurement data obtained in the past and stored at least in the output-side storage unit, selects or generates a measurement voice message datum corresponding to the average, and causes the voice message output unit to output the measurement voice message datum.

13. The measurement system according to any one of claims 1-12, wherein the measurement device includes a temperature measuring unit,
the measurement-side controller causes the measurement-side communication unit to transmit a measured temperature flag generated based on a temperature measured by the temperature measuring unit,
the output-side storage unit stores a plurality of measured temperature voice message data corresponding to a plurality of measured temperature flags,
the output-side controller causes the voice message output unit to output one of the measured temperature voice message data which corresponds to the measured temperature flag transmitted from the measurement-side communication unit.

14. The measurement system according to claim 13, wherein the output-side storage unit stores an improper-environment voice message datum for indicating that environment in which the measurement device is used is improper, and
the output-side controller causes the voice message output unit to output the improper-environment voice message datum depending on content of the measured temperature flag or the frequency.

15. The measurement system according to any one of claims 1-14, wherein the multifunctional portable terminal includes a positional data obtaining unit for obtaining positional information,
the output-side storage unit stores an allowable area datum representing an area where voice message output is allowable and an inhibited area datum representing an area where voice message output should be inhibited,
the output-side controller allows or inhibits voice message output by the voice message output unit based on a result of comparison between the positional information obtained by the positional information obtaining unit and the allowable area datum or the inhibited area datum.
